# EUROPEAN PATENT APPLICATION

(11) **EP 3 903 577 A1**
(43) Date of publication of application: **03.11.2021**
(21) Application number: 19902458.9
(22) Date of filing: 27.12.2019
(51) Int. Cl.: A01H 4/00, A01H 1/08, A01H 1/02

(54) **METHOD FOR GROWING ARUNDO DONAX PLANT**

(30) Priority: 28.12.2018 CN 201811619518; 28.12.2018 CN 201811619497; 28.12.2018 CN 201811619500
(71) Applicant: Wuhan Rundo Biotechnology Co. Ltd, Huangpi Airport Economy Demonstration Zone Wuhan City, Hubei 430300 (CN)
(72) Inventor: DAI, Jun, Wuhan City, Hubei 430300 (CN); HAN, Xiaohong, Wuhan City, Hubei 430300 (CN)
(74) Representative: DREISS Patentanwälte PartG mbB
(86) International application number: PCT/CN2019/129195
(87) International publication number: WO 2020/135712

(57) **Abstract**

A method for Arundo donax haploid plant breeding is provided, which includes: step S1 of collecting flower buds in a late uninucleate stage from a large Arundo donax plant with good stress resistance, then disinfecting the flower buds and extracting anthers under a microscope; step S2: inoculate the anthers obtained in step S1 on a callus culture medium to obtain an Arundo donax callus; step S3: inoculate the Arundo donax callus obtained in step S2 on an adventitious bud differentiation medium to obtain adventitious buds of Arundo donax; step S4 of inoculating the callus of Arundo donax obtained in step S3 on a rooting medium for a rooting culture; and step S5 of determining the ploidy of the product obtained in step S4 to obtain haploid Arundo donax plants.

## Description

### FIELD

The present disclosure relates to the field of biotechnology, in particular to the field of agriculture, and more specifically to a method for plant breeding of Arundo donax.

### BACKGROUND

Arundo donax is a perennial plant in the family Gramineae and the genus Arundo. It has well-developed rhizomes as well as thick, upright, and tough stalks with many nodes and ever-growing branches. It can be used in various industries such as feed, bioethanol production, papermaking, power generation, and environmental restoration.

Existing methods for propagating Arundo donax include seeding, plant-division, cuttings, etc. Generally, the plant-division is the most common method, usually by cutting along the circumference of a plant into a clump with 4-5 buds with a sharp shovel and then transplanting in the early spring. As for this method, the propagation speed is slow, the obtained seedlings tend to carry virus, high labor intensity is required, transportation is inconvenient, causing high afforestation cost, and thus it is difficult to solve the problem of seedling shortage in a short time and it is not conducive to the industrialized production of Arundo donax. When compared to conventional method, the use of tissue culture technology to propagate Arundo donax can obtain numerous consistent and virus-free seedlings in a short period of time, the seedling growth is obviously advantageous, and thus it is an effective way for the industrialized propagation of Arundo donax seedlings.

Polyploid breeding is a valuable method for developing new plant varieties. Polyploid breeding, which began in the early twentieth century, is now widely used in the cultivation of new plant varieties. Polyploids have an increased genome ploidy and, as a result, frequently exhibit massive morphology and enhanced resistance. Triploids, for example, have sterility and can be used as permanent hybrids, as well as being conducive to commercial protection. Triploids with the dual advantages of polyploid and hybrid can be obtained through genome triploidization, demonstrating the benefits of massive morphology and irreplaceability.

However, current Arundo donax plant cultivation methods need to be improved.

### SUMMARY

The current disclosure aims to solve, at least in part, one of the technical problems in the related art. As a result, one goal of the current disclosure is to propose a method for Arundo donax breeding using Androgenesis that improves stress resistance and shortens cultivation time.

The current discovery, in particular, provides the following technical solutions.

According to a first aspect of the present disclosure, the present disclosure provides a method for haploid plant breeding of Arundo donax. According to an embodiment of the present disclosure, the method includes the following steps:
step S1, collecting flower buds in a late uninucleate stage from a large Arundo donax plant with good stress resistance, then performing disinfection treatment, and extracting anthers from the disinfected flower buds under a microscope;
step S2, inoculating the anthers obtained in the step S1 on a callus culture medium for callus induction culture to obtain Arundo donax callus. The callus culture medium is composed of half strength Murashige and Skoog medium (½ MS) supplemented with 0.05-0.15 mg/L of Naphthaleneacetic acid (NAA), 6-8 g/L of sucrose, 5-10 g/L of coconut milk, and optional 2-4 mg/L of 6-benzylaminopurine (6-BA); and when the concentration of the naphthylacetic acid (NAA) is 0.05-0.1 mg/L, the 1/2 MS culture medium contains 2-4 mg/L of 6-benzylaminopurine (6-BA);
step S3, inoculating the Arundo donax callus from step S2 on an adventitious bud differentiation medium for differentiation culture to obtain adventitious Arundo donax buds. The adventitious bud differentiation medium is composed of a ½ MS medium supplemented with 0.1-0.15 mg/L of NAA, 0.8-1.2 mg/L of cytokinin (CPPU), 5-10 g/L of coconut milk, 0.1-0.15 mg/L of 6-BA, and 6-8 g/L of sucrose;
step S4, inoculating the adventitious buds of Arundo donax obtained in the step S3 on a rooting medium for rooting culture. The rooting medium is composed of MS medium supplemented with 0.1-0.15 mg/L of NAA, 6-8 g/L of sucrose, 5-10 g/L of coconut milk, 0.5-1.5 g/L of sepiolite, and 0.8-2.0 g/L of activated carbon; and
step S5, identifying the ploidy of the product obtained in step S4 to obtain haploid Arundo donax plants.

According to an embodiment of the present disclosure, the above method may have any one of the following additional technical features:
According to an embodiment of the present disclosure, the disinfection treatment in step S1 includes washing with sterile water, performing soaking in alcohol for 20-40 min, and performing surface sterilization on the flower buds with sodium hypochlorite, performing rinsing with sterile water, and removing surface moisture from the flower buds, in this order;
According to an embodiment of the present disclosure, in the step S2, the callus induction culture is performed under dark conditions and at a temperature of 25±2 °C.
According to an embodiment of the present disclosure, in the step S3, the differentiation culture is performed under the following conditions: a cultivation temperature of 25±2 °C, with photoperiod of 10-14 h/d, an illumination intensity of 2000-3000 Lux, and a color temperature of 4500-5500 k.
According to an embodiment of the present disclosure, in the step S4, the rooting culture is performed under the following conditions: a culture temperature within a diurnal temperature range of 25-18°C, with photoperiod of 10-14h/d, an illumination intensity of 2000-3000 Lux, and a color temperature of 4500-5500 k.
According to an embodiment of the present disclosure, in the step S1, the large Arundo donax plant with good stress resistance is a triploid, and it is obtained by the following steps:
   a parent selection step including, selecting Arundo donax with good stress resistance and fast growth as a source plant parent;
   a diploid protocorm cultivation step including, aseptically sowing seeds of the source plant parent in a solid culture medium and performing light cultivation to obtain a diploid protocorm, where the solid culture medium is a ½ MS medium supplemented with 0.1-0.15 mg/L of NAA, 6-8 g/L of sucrose, 5-10 g/L of coconut milk, 0.5-1.5 g/L of sepiolite, and 0.8-2.0 g/L of activated carbon.
   steps for preparation of tetraploid Arundo donax seedling, including:
      preparing protoplasts with the diploid protocorm and performing protoplast cell fusion to obtain homologous diploid cells,
      culturing the homologous diploid cells into a tetraploid callus using a culture medium, and
      inducing the tetraploid callus to differentiate, to obtain tetraploid Arundo donax seedlings, where the culture medium for culturing the callus is an MS medium supplemented with 2-4 mg/L of 6-BA, 0.1-0.3 mg/L of IBA, 0.1-0.15 mg/L of NAA, 6-8 g/L of sucrose, 5-10 g/L of coconut milk, and 1-3 mg/L of 2,4-dichlorophenoxyacetic acid (2,4-D);
      a tetraploid Arundo donax seedling cultivation step including, subjecting the tetraploid Arundo donax seedlings to conventional training and acclimation and cultivation until blooming;
      a triploid Arundo donax-obtaining step including, when the tetraploid Arundodonax blooms, performing a sexual hybridization by using the tetraploid Arundo donax as a female parent and a diploid Arundo donax as a male parent, to obtain the hybridized seeds. The hybridized seeds will be seeds of triploid Arundo donax; and
      a triploid Arundo donax cultivation step including, subjecting the seeds of the triploid Arundo donax to asymbiotic germination cultivation, protocorm-like induction, seedling differentiation, seedling transplantation, and cultivation management as a conventional Arundo donax.
According to an embodiment of the present disclosure, in the diploid protocorm cultivation step, the light cultivation is performed under the following conditions: a cultivation temperature within a diurnal temperature range of 25-18°C, a photoperiod of 10-14h/d, an illumination intensity of 2000-3000 Lux, and a color temperature of 4500-5500 k.
According to an embodiment of the present disclosure, in the diploid protocorm cultivation step, a pH of the culture medium ranges from 5 to 6.
According to an embodiment of the present disclosure, in the tetraploid Arundo donax seedling preparation step, the protoplasts are prepared through enzymatic hydrolysis.
According to an embodiment of the present disclosure, in the tetraploid Arundo donax seedling preparation step, the cell fusion is performed by using a cell fusion instrument or a polyethylene glycol (PEG) method for fusion.
According to an embodiment of the present disclosure, in the tetraploid Arundo donax seedling preparation step, the tetraploid callus is identified by conventional dyeing microscopy.
According to an embodiment of the present disclosure, in the step S1, the large Arundo donax plant with good stress resistance is a triploid, and the triploid is obtained by the following steps:
   a triploid breeding source plant selection step including, selecting Arundo donax with good stress resistance and fast growth as a triploid breeding source plant;
   a protocorm breeding step including, aseptically sowing seeds of the triploid breeding source plant in a solid culture medium and performing light cultivation to obtain a protocorm, wherein the solid culture medium is a ½ MS medium supplemented with 0.1-0.15 mg/L of CPPU, 6-8 g/L of sucrose, 5-10 g/L of coconut milk, 0.05-0.1 mg/L of NAA, 0.5-1.5 g/L of sepiolite, 0.5-1.0 g/L of volcanic rock, and 0.8-2.0 g/L of activated carbon;
   a pollen cell population-obtaining step including, aseptically sowing pollens of the triploid breeding source plant in a solid culture medium and performing light cultivation to obtain a pollen cell population, wherein the solid culture medium is a ½ MS medium supplemented with 0.1-0.15 mg/L of CPPU, 6-8 g/L of sucrose, 5-10 g/L of coconut milk, 0.05-0.1 mg/L of NAA, 0.5-1.5 g/L of sepiolite, 0.5-1.0 g/L of volcanic rock, and 0.8-2.0 g/L of activated carbon, at a pH of 5 to 6;
   a protoplast fusion step including, preparing protoplasts from the protocorm and the pollen cell population, and subjecting the obtained protoplasts to cell fusion to obtain fused cells; and
   a triploid seedling breeding step including, subjecting the fused cells to differentiation culture on a solid culture medium to form juvenile plants, identifying triploids, and subjecting triploid plants to asexual tissue cultivation to obtain a large number of triploid seedlings.
According to an embodiment of the present disclosure, in the protocorm breeding step, a pH of the solid culture medium ranges from 5 to 6.
According to an embodiment of the present disclosure, in the protocorm breeding step, the light cultivation is performed under the following conditions: a cultivation temperature within a diurnal temperature range of 25-18°C, a photoperiod of 10-14h/d, an illumination intensity of 2000-3000 Lux, and a color temperature of 4500-5500 k.
According to an embodiment of the present disclosure, in the pollen cell population-obtaining step, the light cultivation is performed under the following conditions: a cultivation temperature within a diurnal temperature range of 25-18°C, a photoperiod of 10-14h/d, an illumination intensity of 2000-3000 Lux, and a color temperature of 4500-5500 k.
According to an embodiment of the present disclosure, in the protoplast fusion step, the protoplasts are prepared through enzymatic hydrolysis.
According to an embodiment of the present disclosure, in the protoplast fusion step, the cell fusion is performed by using a cell fusion instrument or a PEG method for fusion.
According to an embodiment of the present disclosure, in the triploid seedling breeding step, the solid culture medium for the differentiation culture is an MS medium supplemented with 0.1-0.15 mg/L of 6-BA, 0.8-1.2 mg/L of CPPU, and 0.005-0.01 mg/L of NAA.

Compared with the related art, the present disclosure has the following beneficial effects. In the present disclosure, haploid seedlings are obtained through the anther culture breeding of Arundo donax, the stress resistance of Arundo donax is improved, and the breeding period of Arundo donax is shortened. The culture medium for culturing the callus is 1/2 MS + CPPU (0.05-0.15 mg/L) + sucrose (6-8 g/L) + coconut milk (5-10 g/L) + 2,4-D (2-4 mg/L). The differentiation medium is 1/2 MS + NAA (0.1-0.15 mg/L) + CPPU (0.8-1.2 mg/L) + coconut milk (5-10 g/L) + 6-BA(0.1-0.15 mg/L) + sucrose (6-8 g/L). The rooting medium is MS + NAA(0.1-0.15 mg/L) + sucrose (6-8 g/L) + coconut milk (5-10 g/L) + sepiolite (0.5-1.5 g/L) + activated carbon (0.8-2.0 g/L). The performances of the Arundo donax seedlings obtained through breeding with the above-mentioned media are greatly improved.

In addition, according to another aspect of the present disclosure, the present disclosure further provides a method for triploid Arundo donax breeding. The method includes the following steps:
step S1 of parent selection: selecting Arundo donax with good stress resistance and fast growth as a source plant parent;
step S2 of diploid protocorm breeding: aseptically sowing seeds of the source plant in a solid culture medium and performing light cultivation to obtain a diploid protocorm, where the solid culture medium is formulated as 1/2 MS + NAA (0.1-0.15 mg/L) + sucrose (6-8 g/L) + coconut milk (5-10 g/L) + sepiolite (0.5-1.5 g/L) + activated carbon (0.8-2.0 g/L);
step S3 of fusion differentiation of diploid cells: preparing protoplasts with the protocorm obtained in the step S2, performing cell fusion to obtain homologous diploid cells, performing culturing to form a callus using a culture medium, and inducing the identified tetraploid callus to differentiate to obtain tetraploid Arundo donax seedlings, where the culture medium for culturing the callus is formulated as: MS + 6-BA (2-4 mg/L) + IBA (0.1-0.3 mg/L) + sucrose (6-8 g/L) + coconut milk (5-10 g/L) + 2,4-D (1-3 mg/L);
step S4 of tetraploid seedling cultivation: subjecting the tetraploid seedlings to conventional training and acclimation and cultivation until blooming;
step S5 of obtaining triploid Arundo donax: when the tetraploid Arundo donax blooms, performing sexual hybridization by using the tetraploid Arundo donax as female parent and a diploid Arundo donax as male parent, where the hybridized seeds are the triploid Arundo donax; and
step S6 of triploid Arundo donax cultivation: subjecting the seeds of the triploid Arundo donax to asymbiotic germination cultivation, protocorm-like induction, seedling differentiation, seedling transplantation, and cultivation management as conventional Arundo donax.

Preferably, in the step S2, the light cultivation is performed under the following conditions: a cultivation temperature within a diurnal temperature range of 25-18°C, a photoperiod of 10-14h/d, an illumination intensity of 2000-3000 Lux, and a color temperature of 4500-5500 k.

Preferably, in the step S2, a pH of the culture medium ranges from 5 to 6.

Preferably, in the step S3, the protoplasts are prepared through enzymatic hydrolysis.

Preferably, in the step S3, the cell fusion is performed by using a cell fusion instrument or a polyethylene glycol PEG method for fusion.

Preferably, in the step S3, the tetraploid callus is identified by conventional dyeing microscopy.

Compared with the related art, the present disclosure has the following beneficial effects. In the present disclosure, the protoplasts of diploid Arundo donax are first subjected to the cell fusion, and then cultured into a callus on the culture medium, and then the differentiation is induced to obtain tetraploid seedlings of Arundo donax. Then,the sexual hybridization is performed by using the diploid Arundo donax as male parent and the tetraploid Arundo donax as female parent, so as to obtain the triploid Arundo donax. The obtained triploid Arundo donax has strong stress resistance, and has the dual advantages of polyploid and hybrid.

According to a third aspect of the present disclosure, the present disclosure further provides a method for triploid Arundo donax breeding using somatic cell hybridization. The method includes the following steps:
step S1: selecting Arundo donax with good stress resistance and fast growth as a triploid cultivation source plant;
step S2: aseptically sowing seeds of the triploid cultivation source plant in a solid culture medium and performing light cultivation to finally obtain a protocorm, where the solid culture medium is formulated as: 1/2 MS + NAA (0.1-0.15 mg/L) + sucrose (6-8 g/L) + coconut milk (5-10 g/L) + sepiolite (0.5-1.5 g/L) + volcanic rock (0.5-1.0 g/L) + activated carbon (0.8-2.0 g/L);
step S3: aseptically sowing pollens of the source plant in a solid culture medium and performing light cultivation to obtain a pollen cell population, where the solid culture medium is formulated as: 1/2 MS + NAA (0.1-0.15 mg/L) + sucrose (6-8 g/L) + coconut milk (5-10 g/L) + sepiolite (0.5-1.5 g/L) + volcanic rock (0.5-1.0 g/L) + activated carbon (0.8-2.0 g/L), at a pH of 5-6;
step S4: preparing the protoplasts from the protocorm obtained in the step S2 and the pollen cell population obtained in the step S3, and then subjecting the obtained protoplasts to cell fusion; and
step S5: subjecting the fused cells to differentiation culture to form juvenile plants on a solid culture medium, identifying triploids, and subjecting triploid plants to asexual tissue culture to obtain a large number of triploid seedlings.

Preferably, in the step S2, a pH of the solid culture medium ranges from 5 to 6.

Preferably, in the step S2, the light cultivation is performed under the following conditions: a cultivation temperature within a diurnal temperature range of 25-18 °C, a photoperiod of 10-14 h/d, an illumination intensity of 2000-3000 Lux, and a color temperature of 4500-5500 k.

Preferably, in the step S3, the light cultivation is performed under the following conditions: a cultivation temperature within a diurnal temperature range of 25-18 °C, a photoperiod of 10-14 h/d, an illumination intensity of 2000-3000 Lux, and a color temperature of 4500-5500 k.

Preferably, in the step S4, the protoplasts are prepared through enzymatic hydrolysis.

Preferably, in the step S4, the cell fusion is performed by using a cell fusion instrument or a polyethylene glycol PEG method for fusion.

Preferably, in the step S5, the solid culture medium for the differentiation culture is formulated as: MS + 6-BA (0.1-0.15 mg/L) + CPPU (0.8-1.2 mg/L) + NAA (0.005-0.01 mg/L).

Compared with the related art, the present disclosure has the following beneficial effects. In the present disclosure, the solid culture mediums for aseptically sowing the seeds and pollens of the source plant are both formulated as: ½ MS + NAA (0.1-0.15 mg/L) + sucrose (6-8 g/L) + coconut milk (5-10 g/L) + sepiolite (0.5-1.5 g/L) + volcanic rock (0.5-1.0 g/L) + activated carbon (0.8-2.0 g/L); the solid culture medium for the differentiation culture is formulated as: MS + 6-BA (0.1-0.15 mg/L) + CPPU (0.8-1.2 mg/L) + NAA (0.005-0.01 mg/L). In this way, the obtained triploid Arundo donax seedlings have strong stress resistance, and since the seeds and pollens of the source plant are cultivated on the same culture medium, the phenomenon that the cell fusion success rate is low due to different environments in the process of cell fusion is avoided.

### DESCRIPTION OF EMBODIMENTS

Embodiments of the present disclosure are described in detail below. The embodiments described below are merely illustrative and are intended to explain the present disclosure but should not be construed limiting the present disclosure.

To have a more intuitive understanding of the present disclosure, the terms included in the present disclosure are explained and described below. It should be understood by those skilled in the art that these explanations and descriptions are only for convenience of understanding and should not be regarded as a limitation on the protection scope of the present application.

The terms used herein, such as culture medium, additives, etc., have the meanings commonly used in the field of plant cultivation, and those skilled in the art can understand their meanings through conventional technical manuals, and can obtain them through common commercial purchase.

Regarding the terms used herein for describing traits, for example, the terms such as high stress resistance, fast growth, large plant, etc., those skilled in the art can determine through statistical analysis which have corresponding properties among a batch of plants. For example, a corresponding property such as plant size is quantified and ranked, and the top 10% plants are selected as the standard for large plant.

### Example 1

The present disclosure provides a method for Arundo donax breeding using Androgenesis. The method includes the following steps.

S1: Flower buds in a late-uninucleate stage were collected from a large Arundo donax plant with good stress resistance, then a disinfection treatment was performed, and anthers were taken out of the disinfected flower buds under microscope. The disinfection treatment was performed as follows: rinsing the collected flower buds with sterile water, soaking in alcohol for 20 min, followed by surface sterilization on the flower buds with sodium hypochlorite, rinsing again with sterile water, and removing surface moisture of flower buds by absorption.

S2: The anthers obtained in step S1 were inoculated on a callus culture medium for callus induction. The callus culture medium was formulated as: ½ MS + NAA (0.1 mg/L) + 6-BA (2 mg/L). The cultivation was performed under dark conditions and at a temperature of 25±2 °C.

S3: The calli of Arundo donax obtained in step S2 were inoculated on an adventitious bud differentiation medium to obtain adventitious buds of Arundo donax. The adventitious bud differentiation medium was formulated as: ½ MS + NAA (0.1 mg/L) + CPPU (0.8 mg/L) + coconut milk (5 g/L) + 6-BA (0.1 mg/L) + sucrose (6 g/L). The differentiation culture was performed under the following conditions: a cultivation temperature of 25±2 °C, a photoperiod of 10h/d, an illumination intensity of 2000 Lux, and a color temperature of 4500 k.

S4: The adventitious buds of Arundo donax obtained in step S3 were inoculated on a rooting medium for a rooting culture. The rooting medium was formulated as: MS + NAA (0.1 mg/L) + sucrose (6 g/L) + coconut milk (5 g/L) + sepiolite (0.5 g/L) + activated carbon (0.8 g/L). The rooting culture was performed under the following conditions: a culture temperature within a diurnal temperature range of 25-18 °C, a photoperiod of 10h/d, an illumination intensity of 2000 Lux, and a color temperature of 4500 k.

S5: After a ploidy identification, haploid plants of Arundo donax were obtained.

### Example 2

The present disclosure provides a method for Arundo donax breeding using Androgenesis. The method includes the following steps.

S1: Flower buds in a late uninucleate stage were collected from a large Arundo donax plant with good stress resistance, then a disinfection treatment was performed, and anthers were taken out of the disinfected flower buds under microscope. The disinfection treatment was performed as follows: rinsing the removed flower buds with sterile water, soaking in alcohol for 40 minutes, and performing surface sterilization on the flower buds with sodium hypochlorite, performing rinsing again with sterile water, and removing surface moisture of flower buds by absorption.

S2: the anthers obtained in step S1 were inoculated on a callus culture medium for a callus induction. The callus culture medium was formulated as: ½ MS + NAA (0.15 mg/L) + sucrose (8 g/L) + coconut milk (10 g/L) + 6-BA (4 mg/L). The cultivation was performed under dark conditions and at a temperature of 25±2 °C.

S3: the calli of Arundo donax obtained in step S2 were inoculated on an adventitious bud differentiation medium for differentiation culture to obtain adventitious buds of Arundo donax. The adventitious bud differentiation medium was formulated as: ½ MS + NAA (0.15 mg/L) + CPPU (1.2 mg/L) + coconut milk (10 g/L) + 6-BA (0.15 mg/L) + sucrose (8 g/L). The differentiation culture was performed under the following conditions: a cultivation temperature of 25±2 °C, a photoperiod of 14h/d, an illumination intensity of 3000 Lux, and a color temperature of 5500 k.

S4: the adventitious buds of Arundo donax obtained in step S3 were inoculated on a rooting medium for a rooting culture. The rooting medium was formulated as: MS + NAA (0.15 mg/L) + sucrose (8 g/L) + coconut milk (10 g/L) + sepiolite (1.5 g/L) + activated carbon (2.0 g/L). The rooting culture was performed under the following conditions: a culture temperature within a diurnal temperature range of 25-18 °C, a photoperiod of 14h/d, an illumination intensity of 3000 Lux, and a color temperature of 5500 k.

S5: After a ploidy identification, haploid plants of Arundo donax was obtained.

### Example 3

The present disclosure provides a method for Arundo donax breeding using Androgenesis. The method includes the following steps.

S1: flower buds in a late uninucleate stage were collected from a large Arundo donax plant with good stress resistance, then a disinfection treatment was performed, and anthers were taken out of the disinfected flower buds under microscope. The disinfection treatment was performed as follows: rinsing the removed flower buds with sterile water, soaking in alcohol for 30 minutes, performing surface sterilization on the flower buds with sodium hypochlorite, performing rinsing again with sterile water, and removing surface moisture of the flower buds by absorption.

S2: The anthers obtained in step S1 were inoculated on a callus culture medium for a callus induction culture. The callus culture medium was formulated as: ½ MS + NAA (0.12 mg/L) + sucrose (7 g/L) + coconut milk (8 g/L) + 6-BA (3 mg/L). The cultivation was performed under dark conditions and at a temperature of 25±2 °C.

S3: The calli of Arundo donax obtained in step S2 were inoculated on an adventitious bud differentiation medium for differentiation culture to obtain adventitious buds of Arundo donax. The adventitious bud differentiation medium was formulated as: 1/2 MS + NAA (0.12 mg/L) + CPPU (1.0 mg/L) + coconut milk (8 g/L) + 6-BA (0.12 mg/L) + sucrose (7 g/L). The differentiation culture was performed under the following conditions: a cultivation temperature of 25±2°C, a photoperiod of 12h/d, an illumination intensity of 2500 Lux, and a color temperature of 5000 k.

S4: The adventitious buds of Arundo donax obtained in step S3 were inoculated on a rooting medium for a rooting culture. The rooting medium was formulated as: MS + NAA (0.12 mg/L) + sucrose (7 g/L) + coconut milk (8 g/L) + sepiolite (1.0 g/L) + activated carbon (1.4 g/L). The rooting culture was performed under the following conditions: a culture temperature within a diurnal temperature range of 25-18 °C, a photoperiod of 12h/d, an illumination intensity of 2500 Lux, and a color temperature of 5000 k.

S5: After a ploidy identification, haploid plants of Arundo donax were obtained.

### Example 4

The present disclosure provides a method for Arundo donax breeding using Androgenesis. The method includes the following steps.

S1: Flower buds in a late uninucleate stage were collected from a large Arundo donax plant with good stress resistance, then a disinfection treatment was performed, and anthers were taken out of the disinfected flower buds under microscope. The disinfection treatment was performed as follows: rinsing the removed flower buds with sterile water, soaking in alcohol for 20 minutes, performing surface sterilization on the flower buds with sodium hypochlorite, performing rinsing again with sterile water, and removing surface moisture of the flower buds by absorption.

S2: The anthers obtained in step S1 were inoculated on a callus culture medium for a callus induction culture. The callus culture medium was formulated as: 1/2 MS + NAA (0.1 mg/L) + sucrose (8 g/L) + coconut milk (5 g/L) + 6-BA (4 mg/L). The cultivation was performed under dark conditions and at a temperature of 25±2 °C.

S3: The calli of Arundo donax obtained in step S2 were inoculated on an adventitious bud differentiation medium for differentiation culture to obtain adventitious buds of Arundo donax. The adventitious bud differentiation medium was formulated as: ½ MS + NAA (0.1 mg/L) + CPPU (1.2 mg/L) + coconut milk (10 g/L) + 6-BA (0.12 mg/L) + sucrose (6 g/L). The differentiation culture was performed under the following conditions: a cultivation temperature of 25±2 °C, a photoperiod of 12h/d, an illumination intensity of 2000 Lux, and a color temperature of 5500 k.

S4: The adventitious buds of Arundo donax obtained in step S3 were inoculated on a rooting medium for a rooting culture. The rooting medium was formulated as: MS + NAA (0.1 mg/L) + sucrose (6 g/L) + coconut milk (10 g/L) + sepiolite (0.5 g/L) + activated carbon (0.8 g/L). The rooting culture was performed under the following conditions: a culture temperature within a diurnal temperature range of 25-18°C, a photoperiod of 10h/d, an illumination intensity of 3000 Lux, and a color temperature of 5500 k.

S5: After a ploidy identification, haploid plants of Arundo donax were obtained.

### Example 5

The present disclosure provides a method for Arundo donax breeding using Androgenesis. The steps in the method are as follows:
S1: Flower buds in a late uninucleate stage were collected from a large Arundo donax plant with good stress resistance, then a disinfection treatment was performed, and anthers were taken out of the disinfected flower buds under microscope. The disinfection treatment was performed as follows: rinsing the removed flower buds with sterile water, soaking in alcohol for 30 minutes, performing surface sterilization on the flower buds with sodium hypochlorite, rinsing again with sterile water, and removing surface moisture of the flower buds by absorption.
S2: The anthers obtained in step S1 were inoculated on a callus culture medium for a callus induction culture. The callus culture medium was formulated as: ½ MS + NAA (0.1 mg/L) + sucrose (8 g/L) + coconut milk (8 g/L) + 6-BA (4 mg/L). The cultivation was performed under dark conditions and at a temperature of 25±2 °C.
S3: The calli of Arundo donax obtained in step S2 were inoculated on an adventitious bud differentiation medium for differentiation culture to obtain adventitious buds of Arundo donax. The adventitious bud differentiation medium was formulated as: ½ MS + NAA (0.15 mg/L) + CPPU (1.2 mg/L) + coconut milk (10 g/L) + 6-BA (0.1 mg/L) + sucrose (6 g/L). The differentiation culture was performed under the following conditions: a cultivation temperature of 25±2 °C, a photoperiod of 12 h/d, an illumination intensity of 2000 Lux, and a color temperature of 5000 k.
S4: The adventitious buds of Arundo donax obtained in step S3 were inoculated on a rooting medium for a rooting culture. The rooting medium was formulated as: MS + NAA (0.12 mg/L) + sucrose (7 g/L) + coconut milk (8 g/L) + sepiolite (0.5 g/L) + activated carbon (1.6 g/L). The rooting culture was performed under the following conditions: a culture temperature within a diurnal temperature range of 25-18 °C, a photoperiod of 12 h/d, an illumination intensity of 2000 Lux, and a color temperature of 5500 k.
S5: After a ploidy identification, haploid plants of Arundo donax were obtained.

### Example A

The present disclosure provides a method for triploid Arundo donax breeding. The method includes the following steps.

S1: parent selection: Arundo donax with good stress resistance and fast growth was selected as a source plant parent for polyploid breeding.

S2: diploid protocorm breeding: seeds of the source plant were aseptically sown in a solid culture medium and light cultivation was performed. The light cultivation was performed under the following conditions: a cultivation temperature within a diurnal temperature range of 25-18°C, a photoperiod of 10h/d, an illumination intensity of 2000 Lux, and a color temperature of 4500 k. A protocorm was finally obtained. The solid culture medium was formulated as: 1/2 MS + NAA (0.1 mg/L) + sucrose (6 g/L) + coconut milk (5 g/L) + sepiolite (0.5 g/L) + activated carbon (0.8 g/L), and pH of the culture medium was 5.

S3: fusion differentiation of diploid cells: protoplasts were prepared through enzymatic hydrolysis of the protocorm obtained in step S2 and subjected to cell fusion by using a cell fusion instrument or a polyethylene glycol PEG method to obtain homologous diploid cells, which were cultured into a callus using a culture medium, and the tetraploid callus identified by conventional dyeing microscopy was induced to differentiate, so as to obtain tetraploid Arundo donax seedlings. The culture medium for culturing the callus was formulated as: MS + 6-BA (2 mg/L) + IBA (0.1 mg/L) + sucrose (6 g/L) + coconut milk (5 g/L) + 2,4-D (1 mg/L).

S4: tetraploid seedling cultivation: the tetraploid seedlings were subjected to conventional training and acclimation and cultivation until blooming.

S5: obtaining triploid Arundo donax: when the tetraploid Arundo donax bloomed, the tetraploid Arundo donax was used as female parent and a diploid Arundo donax was used as male parent to perform a sexual hybridization. The hybridized seeds were the triploid Arundo donax.

S6: triploid Arundo donax cultivation: the seeds of the triploid Arundo donax were subjected to asymbiotic germination cultivation, protocorm-like induction, seedling differentiation, seedling transplantation, and cultivation management as a conventional Arundo donax.

### Example B

S1: parent selection: Arundo donax with good stress resistance and fast growth was selected as a source plant parent for polyploid breeding.

S2: diploid protocorm breeding: seeds of the source plant were aseptically sown in a solid culture medium and light cultivation was performed. The light cultivation is performed under the following conditions: a cultivation temperature within a diurnal temperature range of 25-18°C, a photoperiod of 14h/d, an illumination intensity of 3000 Lux, and a color temperature of 5500 k. A protocorm was finally obtained. The solid culture medium was formulated as: 1/2 MS + NAA (0.15 mg/L) + sucrose (8 g/L) + coconut milk (10 g/L) + sepiolite (1.5 g/L) + activated carbon (2.0 g/L), and pH of the culture medium was 6.

S3: fusion differentiation of diploid cells: protoplasts were prepared through enzymatic hydrolysis of the protocorm obtained in step S2 and subjected to cell fusion by using a cell fusion instrument or a polyethylene glycol PEG method to obtain homologous diploid cells, which were cultured into a calli using a culture medium, and the tetraploid callus identified by conventional dyeing microscopy was induced to differentiate, so as to obtain tetraploid Arundo donax seedlings. The culture medium for culturing the callus was formulated as: MS + 6-BA (4 mg/L) + IBA (0.3 mg/L) + sucrose (8 g/L) + coconut milk (10 g/L) + 2,4-D (3 mg/L).

S4: tetraploid seedling cultivation: the tetraploid seedlings were subjected to conventional training and acclimation and cultivation until blooming.

S5: obtaining triploid Arundo donax: when the tetraploid Arundo donax bloomed, the tetraploid Arundo donax was used as female parent and a diploid Arundo donax was used as male parent to perform a sexual hybridization. The hybridized seeds were the triploid Arundo donax.

S6: triploid Arundo donax cultivation: the seeds of the triploid Arundo donax were subjected to asymbiotic germination cultivation, protocorm-like induction, seedling differentiation, seedling transplantation, and cultivation management as conventional Arundo donax.

### Example C

S1: parent selection: Arundo donax with good stress resistance and fast growth was selected as a source plant parent for polyploid breeding.

S2: diploid protocorm breeding: seeds of the source plant were aseptically sown in a solid culture medium and light cultivation was performed. The light cultivation is performed under the following conditions: a cultivation temperature within a diurnal temperature range of 25-18°C, a photoperiod of 12h/d, an illumination intensity of 2500 Lux, and a color temperature of 5000 k. A protocorm was finally obtained. The solid culture medium was formulated as: 1/2 MS + NAA (0.12 mg/L) + sucrose (7 g/L) + coconut milk (8 g/L)+ sepiolite (1.0 g/L) + activated carbon (1.4 g/L), and pH of the culture medium was 5.5.

S3: fusion differentiation of diploid cells: protoplasts were prepared through enzymatic hydrolysis of the protocorm obtained in step S2 and subjected to cell fusion by using a cell fusion instrument or a polyethylene glycol PEG method to obtain homologous diploid cells, which were cultured into callus using a culture medium, and the tetraploid callus identified by conventional dyeing microscopy was induced to differentiate, so as to obtain tetraploid Arundo donax seedlings. The culture medium for culturing the callus was formulated as: MS + 6-BA (2.5 mg/L) + IBA (0.15 mg/L) + sucrose (6-8 g/L) + coconut milk (5-10 g/L) + 2,4-D (3 mg/L).

S4: tetraploid seedling cultivation: the tetraploid seedlings were subjected to conventional training and acclimation and cultivation until blooming.

S5: obtaining triploid Arundo donax: when the tetraploid Arundo donax bloomed, the tetraploid Arundo donax was used as female parent and a diploid Arundo donax was used as male parent to perform a sexual hybridization. The hybridized seeds were the triploid Arundo donax.

S6: triploid Arundo donax cultivation: the seeds of the triploid Arundo donax were subjected to asymbiotic germination cultivation, protocorm-like induction, seedling differentiation, seedling transplantation, and cultivation management as conventional Arundo donax.

### Example D

S1: parent selection: Arundo donax with good stress resistance and fast growth was selected as a source plant parent for polyploid breeding.

S2: diploid protocorm breeding: seeds of the source plant were aseptically sown in a solid culture medium and light cultivation was performed. The light cultivation is performed under the following conditions: a cultivation temperature within a diurnal temperature range of 25-18°C, a photoperiod of 10h/d, an illumination intensity of 3000 Lux, and a color temperature of 5500 k. A protocorm was finally obtained. The solid culture medium was formulated as: 1/2 MS + NAA (0.1 mg/L) + sucrose (8 g/L) + coconut milk (10 g/L) + sepiolite (0.5 g/L) + activated carbon (1.0 g/L), and pH of the culture medium was 5.

S3: fusion differentiation of diploid cells: protoplasts were prepared through enzymatic hydrolysis of the protocorm obtained in step S2 and subjected to cell fusion by using a cell fusion instrument or a polyethylene glycol PEG method to obtain homologous diploid cells, which were cultured into callus using a culture medium, and the tetraploid callus identified by conventional dyeing microscopy was induced to differentiate, so as to obtain tetraploid Arundo donax seedlings. The culture medium for culturing the callus was formulated as: MS + 6-BA (3 mg/L) + IBA (0.3 mg/L) + sucrose (8 g/L) + coconut milk (10 g/L) + 2,4-D (1 mg/L).

S4: tetraploid seedling cultivation: the tetraploid seedlings were subjected to a conventional training and acclimation and cultivation until blooming.

S5: obtaining triploid Arundo donax: when the tetraploid Arundo donax bloomed, the tetraploid Arundo donax was used as female parent and a diploid Arundo donax was used as male parent to perform a sexual hybridization. The hybridized seeds were the triploid Arundo donax.

S6: triploid Arundo donax cultivation: the seeds of the triploid Arundo donax were subjected to asymbiotic germination cultivation, protocorm-like induction, seedling differentiation, seedling transplantation, and cultivation management as conventional Arundo donax.

### Example E

S1: parent selection: Arundo donax with good stress resistance and fast growth was selected as a source plant parent for polyploid breeding.

S2: diploid protocorm breeding: seeds of the source plant were aseptically sown in a solid culture medium and light cultivation was performed. The light cultivation was performed under the following conditions: a cultivation temperature within a diurnal temperature range of 25-18°C, a photoperiod of 12h/d, an illumination intensity of 3000 Lux, and a color temperature of 4500 k. A protocorm was finally obtained. The solid culture medium was formulated as: 1/2 MS + NAA (0.15 mg/L) + sucrose (6 g/L) + coconut milk (10 g/L) + sepiolite (0.5 g/L) + activated carbon (1.5 g/L), and pH of the culture medium was 5.5.

S3: fusion differentiation of diploid cells: protoplasts were prepared through enzymatic hydrolysis of the protocorm obtained in step S2 and subjected to cell fusion by using a cell fusion instrument or a polyethylene glycol PEG method to obtain homologous diploid cells, which were cultured into callus using a culture medium, and the tetraploid callus identified by conventional dyeing microscopy was induced to differentiate, so as to obtain tetraploid Arundo donax seedlings. The culture medium for culturing the callus was formulated as: MS + 6-BA (4 mg/L) + IBA (0.2 mg/L) + sucrose (8 g/L) + coconut milk (5 g/L) + 2,4-D (2 mg/L).

S4: tetraploid seedling cultivation: the tetraploid seedlings were subjected to conventional training and acclimation and cultivation until blooming.

S5: obtaining triploid Arundo donax: when the tetraploid Arundo donax bloomed, the tetraploid Arundo donax was used as female parent and a diploid Arundo donax was used as male parent to perform a sexual hybridization. The hybridized seeds were the triploid Arundo donax.

S6: triploid Arundo donax cultivation: the seeds of the triploid Arundo donax were subjected to asymbiotic germination cultivation, protocorm-like induction, seedling differentiation, seedling transplantation, and cultivation management as conventional Arundo donax.

### Transplanting Experiments

Using Example 3 as a transplanting object, 400 triploid Arundo donax seedlings were transplanted in the field, and then managed according to conventional management manners. A survival rate of the transplanting reached 98.75%.

### Example I

S1: Arundo donax with good stress resistance and fast growth was preferably selected as a source plant for triploid breeding.

S2: seeds of the source plant were aseptically sown in a solid culture medium, and light cultivation was performed under the following conditions: a cultivation temperature within a diurnal temperature range of 25-18°C, a photoperiod of 10h/d, an illumination intensity of 2000 Lux, and a color temperature of 4500 k. A protocorm was finally obtained. The solid culture medium was formulated as: 1/2 MS + NAA (0.1 mg/L) + sucrose (6 g/L) + coconut milk (5 g/L) + sepiolite (0.5 g/L) + volcanic rock (0.5 g/L) + activated carbon (0.8 g/L0, pH5.

S3: pollens of the source plant were aseptically sown in a solid culture medium, and light cultivation was performed under the following conditions: a cultivation temperature within a diurnal temperature range of 25-18°C, a photoperiod of 10h/d, an illumination intensity of 2000 Lux, and a color temperature of 4500 k. A pollen cell population was finally obtained. The solid culture medium was formulated as: 1/2 MS + NAA (0.1 mg/L) + sucrose (6 g/L) + coconut milk (5 g/L) + sepiolite (0.5 g/L) + volcanic rock (0.5 g/L) + activated carbon (0.8 g/L), pH5.

S4: protoplasts were prepared through enzymatic hydrolysis of the protocorm obtained in the step S2 and the pollen cell population obtained in the step S3, and then subjected to cell fusion by using a cell fusion instrument.

S5: the fused cells were cultured to differentiate into juvenile plants on a solid culture medium. The solid culture medium was formulated as: MS + 6-BA (0.1 mg/L) + CPPU (0.8 mg/L) + NAA (0.005 mg/L). Triploids were identified, and the triploid plants were subjected to an asexual tissue cultivation to obtain a large number of triploid seedlings.

### Example II

S1: Arundo donax with good stress resistance and fast growth was preferably selected as a source plant for triploid breeding.

S2: seeds of the source plant were aseptically sown in a solid culture medium, and light cultivation was performed under the following conditions: a cultivation temperature within a diurnal temperature range of 25-18°C, a photoperiod of 14h/d, an illumination intensity of 3000 Lux, and a color temperature of 5500 k. A protocorm was finally obtained. The solid culture medium was formulated as: 1/2 MS + NAA (0.15 mg/L) + sucrose (8 g/L) + coconut milk (10 g/L) + sepiolite (1.5 g/L) + volcanic rock (1.0 g/L) + activated carbon (2.0 g/L, pH6.

S3: pollens of the source plant were aseptically sown in a solid culture medium, and light cultivation was performed under the following conditions: a cultivation temperature within a diurnal temperature range of 25-18°C, a photoperiod of 14h/d, an illumination intensity of 3000 Lux, and a color temperature of 5500 k. A pollen cell population was finally obtained. The solid culture medium was formulated as: 1/2 MS + NAA (0.15 mg/L) + sucrose (8 g/L) + coconut milk (10 g/L) + sepiolite (1.5 g/L) + volcanic rock (1.0 g/L) + activated carbon (2.0 g/L), pH6.

S4: protoplasts were prepared through enzymatic hydrolysis of the protocorm obtained in the step S2 and the pollen cell population obtained in the step S3, and then subjected to cell fusion by using a polyethylene glycol PEG method.

S5: the fused cells were cultured to differentiate into juvenile plants on a solid culture medium. The solid culture medium was formulated as: MS + 6-BA (0.15 mg/L) + CPPU (1.2 mg/L) + NAA (0.01 g/L). Triploids were identified, and the triploid plants were subjected to an asexual tissue cultivation to obtain a large number of triploid seedlings.

### Example III

S1: Arundo donax with good stress resistance and fast growth was preferably selected as a source plant for triploid breeding.

S2: seeds of the source plant were aseptically sown in a solid culture medium, and light cultivation was performed under the following conditions: a cultivation temperature within a diurnal temperature range of 25-18°C, a photoperiod of 12h/d, an illumination intensity of 2500 Lux, and a color temperature of 5000 k. A protocorm was finally obtained. The solid culture medium was formulated as: 1/2 MS + NAA (0.12 mg/L) + sucrose (7 g/L) + coconut milk (8 g/L)+ sepiolite (1.0 g/L) + volcanic rock (0.8 g/L) + activated carbon (1.4 g/L), pH5.5.

S3: pollens of the source plant were aseptically sown in a solid culture medium, and light cultivation was performed under the following conditions: a cultivation temperature within a diurnal temperature range of 25-18°C, a photoperiod of 12h/d, an illumination intensity of 2500 Lux, and a color temperature of 5000 k. A protocorm was finally obtained. The solid culture medium was formulated as: 1/2 MS + NAA (0.12 mg/L) + sucrose (7 g/L) + coconut milk (8 g/L)+ sepiolite (1.0 g/L) + volcanic rock (0.8 g/L) + activated carbon (1.4 g/L), pH5.5.

S4: protoplasts were prepared through enzymatic hydrolysis of the protocorm obtained in the step S2 and the pollen cell population obtained in the step S3, and then subjected to cell fusion by using a cell fusion instrument.

S5: the fused cells were cultured to differentiate into juvenile plants on a solid culture medium.

The solid culture medium was formulated as: MS + 6-BA (0.12 mg/L) + CPPU (1.0 mg/L) + NAA (0.008 mg/L). The triploids were identified, and the triploid plants were subjected to an asexual tissue cultivation to obtain a large number of triploid seedlings.

### Example IV

S1: Arundo donax with good stress resistance and fast growth was preferably selected as a source plant for triploid breeding.

S2: seeds of the source plant were aseptically sown in a solid culture medium, and light cultivation was performed under the following conditions: a cultivation temperature within a diurnal temperature range of 25-18°C, a photoperiod of 10h/d, an illumination intensity of 3000 Lux, and a color temperature of 5500 k. A protocorm was finally obtained. The solid culture medium was formulated as: 1/2 MS + NAA (0.1 mg/L) + sucrose (8 g/L) + coconut milk (10 g/L) + sepiolite (0.5 g/L) + volcanic rock (0.5 g/L) + activated carbon (0.8 g/L), pH6.

S3: pollens of the source plant were aseptically sown in a solid culture medium, and light cultivation was performed under the following conditions: a cultivation temperature within a diurnal temperature range of 25-18°C, a photoperiod of 10h/d, an illumination intensity of 3000 Lux, and a color temperature of 5500 k. A protocorm was finally obtained. The solid culture medium was formulated as: 1/2 MS + NAA (0.1 mg/L) + sucrose (8 g/L) + coconut milk (10 g/L) + sepiolite (0.5 g/L) + volcanic rock (0.5 g/L) + activated carbon (0.8 g/L), pH6.

S4: protoplasts were prepared through enzymatic hydrolysis of the protocorm obtained in the step S2 and the pollen cell population obtained in the step S3, and then subjected to cell fusion by using a polyethylene glycol PEG method.

S5: the fused cells were cultured to differentiate into juvenile plants on a solid culture medium. The solid culture medium was formulated as: MS + 6-BA (0.15 mg/L) + CPPU (1.1 mg/L) + NAA (0.005 mg/L). The triploids were identified, and the triploid plants were subjected to an asexual tissue cultivation to obtain a large number of triploid seedlings.

### Example V

S1: Arundo donax with good stress resistance and fast growth was preferably selected as a source plant for triploid breeding.

S2: seeds of the source plant were aseptically sown in a solid culture medium, and light cultivation was performed under the following conditions: a cultivation temperature within a diurnal temperature range of 25-18°C, a photoperiod of 14h/d, an illumination intensity of 2000 Lux, and a color temperature of 4500 k. A protocorm was finally obtained. The solid culture medium was formulated as: 1/2 MS + NAA (0.12 mg/L) + sucrose (6 g/L) + coconut milk (10 g/L) + sepiolite (1.0 g/L) + volcanic rock (0.8 g/L) + activated carbon (0.8 g/L), pH5.5.

S3: pollens of the source plant were aseptically sown in a solid culture medium, and light cultivation was performed under the following conditions: a cultivation temperature within a diurnal temperature range of 25-18°C, a photoperiod of 14h/d, an illumination intensity of 2000 Lux, and a color temperature of 4500 k. A protocorm was finally obtained. The solid culture medium was formulated as: 1/2 MS + NAA (0.12 mg/L) + sucrose (6 g/L) + coconut milk (10 g/L) + sepiolite (1.0 g/L) + volcanic rock (0.8 g/L) + activated carbon (0.8 g/L), pH5.5.

S4: protoplasts were prepared through enzymatic hydrolysis of the protocorm obtained in the step S2 and the pollen cell population obtained in the step S3, and then subjected to cell fusion by using a cell fusion instrument.

S5: the fused cells were cultured to differentiate into juvenile plants on a solid culture medium. The solid culture medium was formulated as: MS + 6-BA (0.1 mg/L) + CPPU (1.2 mg/L) + NAA (0.005 mg/L). Triploids were identified, and the triploid plants were subjected to an asexual tissue cultivation to obtain a large number of triploid seedlings.

### Transplanting Experiments

Using Example 3 as a transplanting object, 300 triploid Arundo donax seedlings were transplanted in the field, and then managed according to conventional management manners. A survival rate of the transplanting reached 98.67%.

In the description of this specification, the description referring to the terms "an embodiment", "some embodiments", "an example", "specific examples", or "some examples" means that the specific features, structures, materials or characteristics described in conjunction with the embodiment or example are included in at least one embodiment or example of the present disclosure. In this specification, the schematic expression of the above terms does not necessarily refer to the same embodiment or example. Moreover, the described specific features, structures, materials, or characteristics may be combined in any one or more embodiments or examples in any suitable manner. In addition, without contradicting each other, different embodiments or examples and features of different embodiments or examples described in the specification can be combined by those skilled in the art.

Although the embodiments of the present disclosure have been shown and described above, it should be understood that the above-mentioned embodiments are illustrative and shall not be interpreted as limiting the present disclosure, and within the scope of the present disclosure, those skilled in the art can make changes, modifications, replacements and variations to the above embodiments.

## Claims

1. A method for haploid plant breeding of Arundo donax, comprising:
step S1 of collecting flower buds in a late uninucleate stage from a large Arundo donax plant with good stress resistance, then performing disinfection treatment, and extracting anthers from the disinfected flower buds under microscope;
step S2 of inoculating the anthers obtained in the step S1 on a callus culture medium for a callus induction culture to obtain a callus of Arundo donax, wherein the callus culture medium is a ½ MS culture medium supplemented with 0.1-0.15 mg/L of NAA, 6-8 g/L of sucrose, 5-10 g/L of coconut milk, 0.05-0.1 mg/L of naphthylacetic acid, and 2-4 mg/L of 6-BA;
step S3 of inoculating the callus of Arundo donax obtained in the step S2 on an adventitious bud differentiation medium for differentiation culture to obtain adventitious buds of Arundo donax, wherein the adventitious bud differentiation medium is a ½ MS culture medium supplemented with 0.1-0.15 mg/L of NAA, 0.8-1.2 mg/L of CPPU, 5-10 g/L of coconut milk, 0.1- 0.15 mg/L of 6-BA, and 6-8 g/L of sucrose;
step S4 of inoculating the adventitious buds of Arundo donax obtained in the step S3 on a rooting medium for rooting culture, wherein the rooting medium is an MS culture medium supplemented with 0.1-0.15 mg/L of NAA, 6-8 g/L of sucrose, 5-10 g/L of coconut milk, 0.5-1.5 g/L of sepiolite, and 0.8-2.0 g/L of activated carbon; and
step S5 of identifying a ploidy of a product obtained in the step S4, to obtain the haploid plants of Arundo donax.

2. The method according to claim 1, wherein, in the step S1, the disinfection treatment comprises:
rinsing the collected flower buds with sterile water, performing soaking in alcohol for 20-40 min, performing surface sterilization on the flower buds with sodium hypochlorite, performing rinsing with sterile water, and removing surface moisture from the flower buds, in this order.

3. The method according to claim 1, wherein, in the step S2, the callus induction culture is performed under dark conditions and at a temperature of 25±2 °C.

4. The method according to claim 1, wherein, in the step S3, the differentiation culture is performed under the following conditions: a cultivation temperature of 25±2 °C, a photoperiod of 10-14 h/d, an illumination intensity of 2000-3000 Lux, and a color temperature of 4500-5500 k.

5. The method according to claim 1, wherein, in the step S4, the rooting culture is performed under the following conditions: a culture temperature within a diurnal temperature range of 25-18 °C, a photoperiod of 10-14 h/d, an illumination intensity of 2000-3000 Lux, and a color temperature of 4500-5500 k.

6. The method according to claim 1, wherein, in the step S1, the large Arundo donax plant with good stress resistance is a triploid Arundo donax, and the triploid Arundo donax is obtained by the following steps:
a parent selection step comprising selecting Arundo donax with good stress resistance and fast growth as a source plant parent;
a diploid protocorm cultivation step comprising, aseptically sowing seeds of the source plant parent in a solid culture medium and performing light cultivation to obtain a diploid protocorm, wherein the solid culture medium is a ½ MS medium supplemented with 0.1-0.15 mg/L of NAA, 6-8 g/L of sucrose, 5-10 g/L of coconut milk, 0.5-1.5 g/L of sepiolite, and 0.8-2.0 g/L of activated carbon;
steps for preparation of tetraploid Arundo donax seedling, including:
- preparing protoplasts with the diploid protocorm and performing cell fusion of the protoplasts to obtain homologous diploid cells;
- culturing the homologous diploid cells into a tetraploid callus using a culture medium; and
- inducing the tetraploid callus to differentiate to obtain tetraploid Arundo donax seedlings, wherein the culture medium for culturing the callus is an MS medium supplemented with 2-4 mg/L of 6-BA, 0.1-0.3 mg/L of IBA, 6-8 g/L of sucrose, 5-10 g/L of coconut milk, and 1-3 mg/L of 2,4-5D;
a tetraploid Arundo donax seedling cultivation step comprising: subjecting the tetraploid Arundo donax seedlings to conventional training and acclimation and cultivation until blooming;
a triploid Arundo donax-obtaining step comprising, when the tetraploid Arundo donax blooms, performing a sexual hybridization by using the tetraploid Arundo donax as a female parent and a diploid Arundo donax as a male parent, to obtain the hybridized seeds. the hybridized seeds obtained being seeds of triploid Arundo donax; and
a triploid Arundo donax cultivation step comprising, subjecting the seeds of the triploid Arundo donax to asymbiotic germination cultivation, protocorm-like induction, seedling differentiation, seedling transplantation, and cultivation management as a conventional Arundo donax.

7. The method according to claim 6, wherein, in the diploid protocorm cultivation step, the light cultivation is performed under the following conditions: a cultivation temperature within a diurnal temperature range of 25-18 °C, a photoperiod of 10-14 h/d, an illumination intensity of 2000-3000 Lux, and a color temperature of 4500-5500 k.

8. The method according to claim 6, wherein, in the diploid protocorm cultivation step, a pH of the culture medium ranges from 5 to 6.

9. The method according to claim 6, wherein, in the tetraploid Arundo donax seedling preparation step, the protoplasts are prepared through enzymatic hydrolysis.

10. The method according to claim 6, wherein, in the tetraploid Arundo donax seedling preparation step, the cell fusion is performed by using a cell fusion instrument or a polyethylene glycol PEG method.

11. The method according to claim 6, wherein, in the tetraploid Arundo donax seedling preparation step, the tetraploid callus is identified by conventional dyeing microscopy.

12. The method according to claim 1, wherein, in the step S1, the large Arundo donax plant with good stress resistance is a triploid Arundo donax, and the triploid Arundo donax is obtained by the following steps:
a triploid breeding source plant selection step involving, selecting Arundo donax with good stress resistance and rapid growth as a triploid breeding source plant;
a protocorm breeding step comprising, aseptically sowing seeds of the triploid breeding source plant in a solid culture medium and performing light cultivation to obtain a protocorm, wherein the solid culture medium is a ½ MS medium supplemented with 0.1-0.15 mg/L of NAA, 6-8 g/L of sucrose, 5-10 g/L of coconut milk, 0.5-1.5 g/L of sepiolite, 0.5-1.0 g/L of volcanic rock, and 0.8-2.0 g/L of activated carbon;
a step for obtaining pollen cell population that comprises aseptically sowing pollens of the triploid breeding source plant in a solid culture medium and performing light cultivation to obtain a pollen cell population, wherein the solid culture medium is a ½ MS medium supplemented with 0.1-0.15 mg/L of NAA, 6-8 g/L of sucrose, 5-10 g/L of coconut milk, 0.5-1.5 g/L of sepiolite, 0.5-1.0 g/L of volcanic rock, and 0.8-2.0 g/L of activated carbon, at a pH of 5 to 6;
a protoplast fusion step comprising, preparing protoplasts from the protocorm and the pollen cell population and subjecting the obtained protoplasts to cell fusion to obtain fused cells; and
a triploid seedling breeding step comprising: exposing the fused cells to differentiation culture on a solid culture medium to form juvenile plants, identifying triploids, and subjecting triploid plants to asexual tissue cultivation to obtain a large number of triploid seedlings.

13. The method according to claim 12, wherein a pH of the solid culture medium ranges from 5 to 6 during the protocorm breeding step.

14. The method according to claim 12, wherein the light cultivation is performed in the protocorm breeding step under the following conditions: a cultivation temperature within a diurnal temperature range of 25-18°C, a photoperiod of 10-14 h/d, an illumination intensity of 2000-3000 Lux, and a color temperature of 4500-5500 k.

15. The method according to claim 12, wherein in the pollen cell population-obtaining step, the light cultivation is performed under the following conditions: a cultivation temperature within a diurnal temperature range of 25-18°C, a photoperiod of 10-14h/d, an illumination intensity of 2000-3000 Lux, and a color temperature of 4500-5500 k.

16. The method according to claim 12, wherein the protoplasts are prepared by enzymatic hydrolysis during the protoplast fusion step.

17. The method according to claim 12, wherein the cell fusion is performed in the protoplast fusion step using a cell fusion instrument or a polyethylene glycol PEG method for fusion.

18. The method according to claim 12, wherein the solid culture medium for the differentiation culture in the triploid seedling breeding step is an MS medium supplemented with 0.1-0.15 mg/L of 6-BA, 0.8-1.2 mg/L of CPPU, and 0.005-0.01 mg/L of NAA.

19. The method according to claim 1, wherein the callus culture medium a ½ MS culture medium supplemented with 0.05-0.1 mg/L of NAA, 6-8 g/L of sucrose, 5-10 g/L of coconut milk, 0.005-0.1 mg/L of naphthylacetic acid, and 2-4 mg/L of 6-BA.
